# EUROPEAN PATENT SPECIFICATION

(11) **EP 1 868 626 B1**
(45) Date of publication and mention of the grant of the patent: **10.09.2008**
(21) Application number: 06742754.2
(22) Date of filing: 28.04.2006
(51) Int. Cl.: A61K 36/16, A23L 1/29, A61P 25/28, A61P 9/00

(54) **IMPROVED METHOD FOR PREPARING GINKGO EXTRACTS HAVING A REDUCED CONTENT OF POLYCYCLIC AROMATIC HYDROCARBONS**
VERBESSERTES VERFAHREN ZUR HERSTELLUNG VON GINKGOEXTRAKTEN MIT GERINGEM GEHALT AN POLYCYCLISCHEN AROMATISCHEN KOHLENWASSERSTOFFEN
PROCEDE AMELIORE DE PREPARATION D'EXTRAITS DE GINKGO A TENEUR REDUITE EN HYDROCARBURES AROMATIQUES POLYCYCLIQUES

(30) Priority: 03.05.2005 DE 102005020640
(43) Date of publication of application: 26.12.2007
(73) Proprietor: Dr. Willmar Schwabe GmbH & Co. KG, 76227 Karlsruhe (DE)
(72) Inventor: HAUER, Hermann, 76228 Karlsruhe (DE); OSCHMANN, Rainer, 76829 Landau (DE); WAIMER, Frank, 76228 Karlsruhe (DE)
(74) Representative: Adam, Holger
(86) International application number: PCT/EP2006/004030
(87) International publication number: WO 2006/117170

(56) References cited:
- EP-A- 0 431 535
- "EU food contaminant laws - responsibility of ingredient makers by Peter Berry Ottaway" NUTRACEUTICALS INTERNATIONAL, vol. 8, no. 8, August 2003 (2003-08), pages 10-11, XP009070438 ISSN: 1362-4511
- ANONYMOUS: "PAHs IN DIETARY SUPPLEMENTS" INTERNET ARTICLE, [Online] December 2005 (2005-12), XP002393109 Retrieved from the Internet: URL:http://www.food.gov.uk/multimedia/pdfs /fsis8605.pdf> [retrieved on 2006-08-02]

## Description

The present invention relates to an improved multi-step method for preparing an extract from Ginkgo biloba having a reduced content of polycyclic aromatic hydrocarbons.

Since decades, extracts from the leaves of Ginkgo biloba are used as a medicament. They are currently used for the treatment of different kinds of dementia and symptoms thereof as well as cerebral and peripheral blood circulation disorders. Ingredients, the efficacy is associated with, are terpene lactones (ginkgolides A, B, C und bilobalide) as well as glycoside and flavones (quercetin, kaempferol and isorhamnetin). However, the leaves of Ginkgo biloba also contain considerable amounts of components which do not contribute to the desired efficacy, but which may be responsible for risks and side effects. These are particularly unpolar plant ingredients such as ginkgolic acids and unpolar impurities due to environmental influences such as polycyclic aromatic hydrocarbons (PAHs). In a Ginkgo extract which is efficacious and at the same time as safe as possible and as low in side effects as possible, these compounds should thus not be present to the largest possible extent.

Due to the existing or increasing atmospheric pollution in large parts of the world, which is a consequence of the rapidly increasing consumption of fossil fuels such as petroleum and which also concerns growing areas of Ginkgo biloba, Ginkgo leaves are recently provided to an increasing extent, which are polluted with considerable amounts of unpolar impurities due to environmental influences, particularly polycyclic aromatic hydrocarbons (PAHs). In this regard, PAHs are a general term for aromatic compounds having fused ring systems such as fluorene, phenanthrene, anthracene, fluoranthene, pyrene, benz[a]anthracene, chrysene, benzo[b]fluoranthene, benzo[k]fluoranthene, benzo[a]pyrene, indeno[1,2,3-cd]pyrene, dibenzo[ah]anthracene and benzo[ghi]perylene. At least a part of the PAHs are carcinogenic such that there is a significant necessity to ensure that extracts produced from polluted Ginkgo leaves are set free from these pollutants to the largest possible extent. Generally, in the case of carcinogenic substances, a lower limit, under which PAHs are considered to be harmless, cannot be defined.

A Ginkgo extract which has a low content of Ginkgolic acid (< 10 ppm and < 1 ppm, respectively) is already described in EP 431535 B1. However, it is not described whether this method is simultaneously capable of largely depleting PAHs present in Ginkgo leaves. It has now been found that the method according to EP 431535 B1 already leads to a PAH depletion. Since there is no limit of harmlessness, there is still a significant necessity to further improve this method such that a higher depletion of PAHs occurs.

Therefore, it is the object underlying the present invention to modify the method described in EP 431535 B1 such that the contents of PAHs are further minimized.

According to claim 3 of EP 431535 B1 (the preferred parameters of claim 4 depending thereon are given in parenthesis), the total disclosure content of which shall be explicitly incorporated into the present application by reference, the method for preparing an extract from Ginkgo biloba leaves claimed therein is characterized in that
(a) fresh or dried green leaves of Ginkgo biloba are extracted at a temperature of about 40 to 100°C using aqueous acetone, an aqueous alkanol having 1 to 3 carbon atoms or anhydrous methanol,
(b) the organic solvent is largely separated from the extract to a maximum content of 10% by weight (maximum content of 5% by weight), wherein water may be added during the final distillation steps,
(c) the remaining concentrated aqueous solution is diluted with water to a solids content of 5 to 25% by weight (15 to 20% by weight), followed by cooling to a temperature below 25 °C (cooled to a temperature of about 10 to 12°C) and allowing to stand until a precipitate is formed, and the resulting precipitate, which consists of the lipophilic components that are not well soluble in water, is removed,
(d) ammonium sulfate is added to the remaining aqueous solution (to a content of 30% by weight) and the solution formed is extracted with methyl ethyl ketone or a mixture of methyl ethyl ketone and acetone (in a ratio of 9:1 to 4:6, preferably 6:4),
(e) the extract obtained is concentrated to a solids content of 50 to 70% and the concentrate thus obtained is diluted with water such that a solution containing 50% by weight water and 50% by weight ethanol at a solids content of 10% by weight is obtained,
(f) an aqueous solution of a lead salt (lead acetate, lead hydroxide acetate or lead nitrate or an aqueous suspension of lead hydroxide) is added to the solution thus obtained until a change in colour from brown to amber occurs, and the precipitate formed is removed, or a polyamide is used instead of the lead salt,
(g) the remaining aqueous-alcoholic solution is extracted with an aliphatic or cycloaliphatic solvent having a boiling point of 60 to 100°C in order to further remove the alkylphenol compounds,
(h) the remaining aqueous-alcoholic solution is concentrated under reduced pressure to an ethanol content of about 5% and ammonium sulfate is added to a content of 20% by weight,
(i) the solution obtained is extracted with a mixture of methyl ethyl ketone and ethanol in a ratio of 8:2 to 5:5, preferably 6:4,
(k) the resulting organic phase is concentrated to a solids content of 50 to 70% by weight,
(l) the resulting concentrate is concentrated under reduced pressure at a maximum temperature of 60 to 80°C, thereby obtaining a dry extract having a water content of less than 5%.

It has now surprisingly been found that a better depletion of PAHs can be achieved by a combination of several modifications of the method according to EP 431535 B1 than it is obtained when example 1 of EP 431535 B1 is reproduced.

According to the present invention, the following modifications contribute to a more effective PAH depletion:
- in step (b), the organic solvent is separated to a maximum content of 2% by weight, preferably 1 % by weight,
- in step (c) cooling is carried out to ≤ 6°C,
- in step (c) the period for the precipitate formation is additionally extended to at least 1 hour, preferably to at least 10 hours at ≤ 6°C,
- after step (e) a filtration step is incorporated and the filtrate is further processed,
- in step (g) heptane is employed as the aliphatic or cycloaliphatic solvent,
- in step (g) the extraction using heptane is carried out at least five times,
- in step (k) drying is carried out with up to 20% by weight ammonium sulfate prior to concentrating,
- after step (k) ethanol is added such that an ethanol content of at least 80% by weight results and the temperature of up to 12°C is maintained for 2 to 10 h and a filtration is carried out.

Thus, the method according to the present invention for preparing an extract from Ginkgo biloba leaves is characterized in that
(a) fresh or dried green leaves of Ginkgo biloba (drug) are extracted at a temperature of about 40 to 100°C, preferably 40 to 60°C with aqueous acetone having a content of 20-90% by weight, an aqueous alkanol having 1 to 3 carbon atoms and a content of 20-90% by weight (methanol, ethanol, n-propanol, isopropanol) or anhydrous methanol, wherein the drug-to-solvent ratio is 1:4 to 1:20, preferably 1:5 to 1:10,
(b) the organic solvent is largely separated from the extract to a maximum content of 2% by weight, preferably 1 % by weight, wherein water may be added during the final distillation steps,
(c) the remaining concentrated aqueous solution is diluted with water to a solids content of 5 to 25% by weight, cooled under agitation to a temperature below 6°C and allowed to stand for at least 1 hour, preferably for at least 10 hours at this temperature and the resulting precipitate consisting of lipophilic components which are not well soluble in water, is removed,
(d) ammonium sulfate (preferably about 30% by weight) is added to the remaining aqueous solution and the solution formed is extracted with methyl ethyl ketone or a mixture of methyl ethyl ketone and acetone (in a ratio of preferably 6:4),
(e) the extract obtained is concentrated to a solids content of 50 to 70% and the concentrate thus obtained is diluted with water and ethanol such that a solution containing 50% by weight water and 50% by weight ethanol at a solids content of 10% is obtained,
   a filtration is carried out and the filtrate is further processed in (f),
(f) an aqueous solution of a lead salt (preferably lead hydroxide acetate) is added to the solution thus obtained, until a change in colour from brown to amber occurs, and the precipitate formed is removed, or a polyamide is used instead of the lead salt,
(g) the remaining aqueous alcoholic solution is extracted with heptane, wherein the extraction is carried out at least five times in order to further remove the alkylphenol compounds,
(h) the remaining aqueous alcoholic solution is concentrated to an ethanol content of about 5% under reduced pressure and ammonium sulfate is added to a content of 20% by weight,
(i) the solution obtained is extracted with a mixture of methyl ethyl ketone and ethanol in a ratio of 8:2 to 5:5, preferably 6:4,
(k) the resulting organic phase is dried with ≤ 20% by weight ammonium sulfate and concentrated to a solids content of 50 to 70% by weight,
   added with ethanol such that an ethanol content of at least 80% by weight is obtained, and maintained for at least 2h, preferably up to 10h at ≤ 12°C and filtered,
(I) the resulting filtrate is concentrated under reduced pressure at a maximum temperature of 60 to 80°C and dried, thereby obtaining a dry extract having a water content of less than 5%.

The preferred extraction solvent in step (a) is aqueous acetone, particularly preferred with an acetone content of about 60% by weight.

According to the European Pharmacopoeia dry extracts generally have a dry residue of at least 95% by weight.

The extracts obtained according to the present invention can be administered in the form of powders, granules, tablets, dragées (coated tablets) or capsules, preferably orally. In order to prepare tablets, the extract is mixed with suitable pharmaceutically acceptable adjuvants such as lactose, cellulose, silicon dioxide, croscarmellose and magnesium stearate and pressed into tablets which are optionally provided with a suitable coating made of, for example, hydroxymethylcellulose, polyethyleneglycol, pigments (such as titanium dioxide, iron oxide) and talcum. The extract obtained according to the present invention can also be filled into capsules, optionally under the addition of adjuvants such as stabilizers, fillers and the like. The dosage is such that 10 to 2000 mg, preferably 50 to 1000 mg and particularly preferred 100 to 500 mg extract are administered per day.

Furthermore, medicaments, food products and other preparations, which contain these extracts, optionally in combination with other substances such as active ingredients and/or pharmaceutically acceptable adjuvants can be obtained. The term "food product" as used herein particularly refers to dietetic food products, dietary supplement products as well as medical food and dietary supplement.

### Examples

### Comparative Example 1

Dried and ground leaves of Ginkgo biloba with a PAH contamination due to environmental influences were extracted twice at a temperature of about 58°C using each time 7.5 times their weight (w/w) made up of acetone/water 60/40 (w/w) (step a)).
The organic solvent was largely separated from the combined extract solutions, wherein water was added (solids content: about 15% by weight; acetone content: 2.51 %; step b)). The product was cooled to a temperature of about 12°C under agitation and after one hour the resulting precipitate is removed (step c)).
About 30% by weight ammonium sulfate was added to the remaining aqueous solution and the solution formed was extracted with a mixture of methyl ethyl ketone and acetone in a ratio of 6:4 (w/w) (step d)).
The extract thus obtained was concentrated to a solids content of about 60% by weight and the concentrate thus obtained was diluted with water and ethanol such that a solution containing 50% by weight water and 50% by weight ethanol at a solids content of about 10% was obtained (step e)). This solution was added with an aqueous solution of lead hydroxide acetate and the precipitate formed was separated (step f)).
The remaining aqueous alcoholic solution was extracted three times using each time 1/3 of its volume made up of hexane (step g)).
Then the remaining aqueous alcoholic solution was concentrated under reduced pressure (ethanol content about 5%) and about 20% by weight ammonium sulfate was added (step h)).
The solution obtained was extracted with a mixture of methyl ethyl ketone and ethanol in a ratio of 6:4 (w/w) (step i)).
The resulting organic phase was dried with about 20% by weight ammonium sulfate and concentrated to a solids content of about 60% by weight (step k)). The concentrate was freeze-dried (step I)).

### Example 1:

Dried and ground leaves of Ginkgo biloba with a PAH contamination due to environmental influences (from the same batch as in Comparative Example 1) were extracted twice using each time 7.5 times their weight (w/w) made up of acetone/water 60/40 (w/w) at a temperature of about 58°C (step a)).
The organic solvent was largely removed from the combined extract solution, wherein water was added (solids content: about 15% by weight; acetone content < 0.01 %) (step b)).
The solution was cooled to a temperature of about 4°C under agitation and after one hour the precipitate formed was removed (step c)).
About 30% by weight ammonium sulfate was added to the remaining aqueous solution and the solution formed was extracted with a mixture of methyl ethyl ketone and acetone in a ratio of 6:4 (w/w) (step d)).
The extract obtained was concentrated to a solids content of about 60% by weight and the concentrate thus obtained was diluted with water and ethanol such that a solution containing 50% by weight water and 50% by weight ethanol at a solids content of about 10% by weight was obtained. The solution was filtered (step e)), the filtrate was added with an aqueous solution of lead hydroxide acetate and the precipitate formed was separated (step f)). The remaining aqueous alcoholic solution was extracted three times using each time 1/3 of its volume made up of heptane (step g)).
Then the remaining aqueous alcoholic solution was concentrated under reduced pressure (ethanol content of about 5%) and about 20% by weight ammonium sulfate was added (step h)).
The solution obtained was extracted with a mixture of methyl ethyl ketone and ethanol in a ratio of 6:4 (w/w) (step i)).
The resulting organic phase was dried with about 20% by weight ammonium sulfate and concentrated to a solids content of about 60% by weight. The concentrate was added with ethanol such that an ethanol content of at least 80% by weight was obtained. The product was cooled to 10°C for five hours, filtered (step k)) and freeze-dried (step I)).

### Example 2:

Dried and ground leaves of Ginkgo biloba with a PAH contamination due to environmental influences (from the same batch as in Comparative Example 1) were extracted twice at a temperature of about 58°C using each time 7.5 times their weight (w/w) made up of acetone/water 60/40 (w/w) (step a)).
The organic solvent was largely separated from the combined extract solutions, wherein water was added (solids content: about 15% by weight; acetone content: 0.01 %, step b)).
The product was cooled to a temperature of about 4°C under agitation and after about 15 h the resulting precipitate was removed (step c)).
About 30% by weight ammonium sulfate was added to the remaining aqueous solution and the solution formed was extracted with a mixture of methyl ethyl ketone and acetone in a ratio of 6:4 (w/w) (step d)).
The extract obtained was concentrated to a solids content of about 60% by weight and the concentrate thus obtained was diluted with water and ethanol such that a solution containing 50% by weight water and 50% by weight ethanol at a solids content of about 10% by weight was obtained. The solution was filtered (step e)), the filtrate was added with an aqueous solution of lead hydroxide acetate and the precipitate formed was separated (step f)).
The remaining aqueous alcoholic solution was extracted five times using each time 1/3 of its volume made up of heptane (step g)).
Then the remaining aqueous alcoholic solution was concentrated under reduced pressure (ethanol content of about 5%) and about 20% by weight ammonium sulfate was added (step h)).
The solution obtained was extracted with a mixture of methyl ethyl ketone and ethanol in a ratio of 6:4 (w/w) (step i)).
The resulting organic phase was dried with about 20% by weight ammonium sulfate and concentrated to a solids content of about 60% by weight. The concentrate was added with ethanol, such that an ethanol content of at least 80% by weight was obtained. The product was cooled to 10°C for five hours, filtered (step k)) and freeze-dried (step I)).

### Results:

The contents of the polycyclic aromatic hydrocarbons (PAH) fluorene, fluoranthene, pyrene and benzo[k]fluoranthene in the resulting extracts can be seen from the following table. It is found that the sum of the contents in Examples 1 and 2 according to the present invention is significantly lower than in Comparative Example 1. Furthermore, a still lower value is found in Example 2 than in Example 1 due to additional modifications of the method.

**Table 1: Compositions of the extracts according to the above examples**

| Extract according to | Comparative Example 1 | Example 1 | Example 2 |
|---|---|---|---|
| acetone content (step b)) | 2.51 % | < 0.01 % | 0.01 % |
| temperature (step c)) | 12 °C | 4°C | 4°C |
| time of precipitation (step c)) | 1 h | 1 h | about 15 h |
| Filtration (step e)) | no | yes | yes |
| Extraction solvent (step g)) | Hexane | Heptane | Heptane |
| Extraction (step g)) | 3 times with 1/3 vol. | 3 times with 1/3 vol. | 5 times with 1/3 vol. |
| Precipitation (step k)) | no | yes | yes |
| Fluorene [ppb] | 1.6 | 1.9 | 1.9 |
| Fluoranthene [ppb] | 3.2 | 3.5 | 2.4 |
| Pyrene [ppb] | 10 | 2.0 | 1.9 |
| Benzo[k]fluoranthene [ppb] | 1.7 | n.d. (< 0.5) | n.d. (< 0.5) |
| Total amount of PAHs [ppb] | 16.5 | 7.4 | 6.2 |

| | | | |
|---|---|---|---|
| n.d. = not detectable | | | |

The PAHs phenanthrene, anthracene, benz[a]anthracene, chrysene, benzo[b]fluoranthene, benzo[a]pyrene, indeno[1,2,3-cd]pyrene, dibenzo[ah]anthracene and benzo[ghi]perylene which are not cited in the table, were not detectable in the extracts according to Comparative Example 1 and Examples 1 and 2 (content < 0.5 ppb).

## Claims

1. Method for preparing an extract from Ginkgo biloba having a reduced content of polycyclic aromatic hydrocarbons, **characterized in that**
(a) fresh or dried green leaves of Ginkgo biloba (drug) are extracted at a temperature of about 40 to 100°C using aqueous acetone, an aqueous alkanol having 1 to 3 carbon atoms or anhydrous methanol,
(b) the organic solvent is largely separated from the extract to a maximum content of 2% by weight, wherein water may be added during the final distillation steps,
(c) the remaining concentrated aqueous solution is diluted with water to a solids content of 5 to 25% by weight, cooled under agitation to a temperature below 6°C, allowed to stand for at least 1 hour at this temperature and the resulting precipitate consisting of the lipophilic components which are not well soluble in water, is removed,
(d) ammonium sulfate is added to the remaining aqueous solution and the solution formed is extracted with methyl ethyl ketone or a mixture of methyl ethyl ketone and acetone,
(e) the extract obtained is concentrated to a solids content of 50 to 70% by weight and the concentrate thus obtained is diluted with water and ethanol such that a solution containing 50% by weight water and 50% by weight ethanol at a solids content of about 10% by weight is obtained,
a filtration is carried out and the filtrate is further processed in (f),
(f) an aqueous solution of a lead salt is added to the solution thus obtained until a change in colour from brown to amber occurs, and the precipitate formed is removed, or a polyamide is used instead of the lead salt,
(g) the remaining aqueous alcoholic solution is extracted with heptane in order to further remove the alkylphenol compounds,
(h) the remaining aqueous alcoholic solution is concentrated under reduced pressure to an ethanol content of about 5% by weight and added with ammonium sulfate to a content of 20% by weight,
(i) the solution obtained is extracted with a mixture of methyl ethyl ketone and ethanol in a ratio of 8:2 to 5:5 (w/w),
(k) the resulting organic phase is dried with ≤ 20% by weight ammonium sulfate and concentrated to a solids content of 50 to 70% by weight, added with ethanol such that an ethanol content of at least 80% by weight is obtained, maintained for at least 2 h at ≤ 12°C and filtered,
(l) the resulting filtrate is concentrated under reduced pressure at a maximum temperature of 60 to 80°C and dried, thereby obtaining a dry extract having a water content of less than 5% by weight.

2. Method according to claim 1, **characterized in that** in step (a) the temperature is 40 to 60°C and/or the drug-to-solvent ratio is 1:4 to 1:20.

3. Method according to claim 1 or 2, **characterized in that** in step (b) the organic solvent is separated from the extract to a maximum content of 1% by weight.

4. Method according to any one of claims 1 to 3, **characterized in that** in step (c) allowing to stand is carried out for at least 10 hours at below 6°C.

5. Method according to any one of claims 1 to 4, **characterized in that** in step (d) about 30% by weight ammonium sulfate is added to the remaining aqueous solution and/or the solution formed is extracted with a mixture of methyl ethyl ketone and acetone in a ratio of 6:4 (w/w).

6. Method according to any one of claims 1 to 5, **characterized in that** in the lead salt in step (f) is lead hydroxide acetate.

7. Method according to any one of claims 1 to 6, **characterized in that** the extraction in step (g) is carried out at least five times.

8. Method according to any one of claims 1 to 7, **characterized in that** in step (i) the solution obtained is extracted with a mixture of methyl ethyl ketone and ethanol in a ratio of 6:4 (w/w).

## Patentansprüche

1. Verfahren zur Herstellung eines Extrakts aus Ginkgo biloba mit vermindertem Gehalt an polycyclischen aromatischen Kohlenwasserstoffen, **dadurch gekennzeichnet, dass**
(a) frische oder getrocknete grüne Blätter von Ginkgo biloba (Droge) bei einer Temperatur von etwa 40 bis 100 °C mit wässrigem Aceton, einem wässrigen Alkanol mit 1 bis 3 Kohlenstoffatomen oder wasserfreiem Methanol extrahiert werden,
(b) das organische Lösungsmittel aus dem Extrakt größtenteils bis zu einem maximalen Gehalt von 2 Gew.-% abgetrennt wird, wobei in den letzten Destillationsstufen Wasser zugefügt werden kann,
(c) die verbliebene konzentrierte wässrige Lösung mit Wasser bis zu einem Feststoffgehalt von 5 bis 25 Gew.-% verdünnt, unter Rühren bis auf eine Temperatur unter 6 °C gekühlt, mindestens 1 Stunde bei dieser Temperatur stehengelassen wird, und der resultierende Niederschlag, der aus den lipophilen Bestandteilen besteht, die sich in Wasser nicht gut lösen, entfernt wird,
(d) Ammoniumsulfat zur verbliebenen wässrigen Lösung zugefügt und die entstandene Lösung mit oder einem Gemisch aus Methylethylketon und Aceton extrahiert wird,
(e) der erhaltene Extrakt auf einen Feststoffgehalt von 50 bis 70 Gew.-% konzentriert wird und das so erhaltene Konzentrat mit Wasser und Ethanol so verdünnt wird, dass eine Lösung erhalten wird, die 50 Gew.-% Wasser und 50 Gew.-% Ethanol bei einem Feststoffgehalt von etwa 10 Gew.-% enthält, eine Filtration durchgeführt und das Filtrat in (f) weiterverarbeitet wird,
(f) eine wässrige Lösung eines Bleisalzes zu der auf diese Weise erhaltenen Lösung solange zugefügt wird, bis eine Farbänderung von Braun zu Umbra eintritt, und der entstandene Niederschlag entfernt wird, oder ein Polyamid anstelle eines Bleisalzes verwendet wird,
(g) die verbliebene wässrig-alkoholische Lösung mit Heptan extrahiert wird, um die Alkylphenolverbindungen weiter zu entfernen,
(h) die verbliebene wässrig-alkoholische Lösung unter vermindertem Druck auf einen Ethanolgehalt von etwa 5 Gew.-% konzentriert und Ammoniumsulfat bis zu einem Gehalt von 20 Gew.-% zugefügt wird,
(i) die erhaltene Lösung mit einem Gemisch aus Methylethylketon und Ethanol in einem Verhältnis von 8:2 bis 5:5 (G/G) extrahiert wird,
(k) die resultierende organische Phase mit ≤ 20 Gew.-% Ammoniumsulfat getrocknet und zu einem Feststoffgehalt von 50 bis 70 Gew.-% konzentriert wird,
so mit Ethanol versetzt wird, dass ein Ethanol-Gehalt von mindestens 80 Gew.-% resultiert, und für mindestens 2 h bei ≤ 12 °C gehalten und filtriert wird,
(l) das resultierende Filtrat unter vermindertem Druck bei einer Höchsttemperatur von 60 bis 80 °C konzentriert und getrocknet wird, wobei ein Trockenextrakt mit einem Wassergehalt von weniger als 5 Gew.-% erhalten wird.

2. Verfahren nach Anspruch 1, **dadurch gekennzeichnet, dass** in Schritt (a) die Temperatur 40 bis 60 °C beträgt und/oder das Droge-zu-Lösungsmittel-Verhältnis 1:4 bis 1:20 ist.

3. Verfahren nach Anspruch 1 oder 2, **dadurch gekennzeichnet, dass** in Schritt (b) das organische Lösungsmittel aus dem Extrakt bis zu einem maximalen Gehalt von 1 Gew.-% abgetrennt wird.

4. Verfahren nach einem der Ansprüche 1 bis 3, **dadurch gekennzeichnet, dass** in Schritt (c) für mindestens etwa 10 Stunden bei unter 6 °C stehengelassen wird.

5. Verfahren nach einem der Ansprüche 1 bis 4, **dadurch gekennzeichnet, dass** in Schritt (d) etwa 30 Gew.-% Ammoniumsulfat zu der verbliebenen wässrigen Lösung gegeben werden und/oder die gebildete Lösung mit einem Gemisch aus Methylethylketon und Aceton in einem Verhältnis von 6:4 (G/G) extrahiert wird.

6. Verfahren nach einem der Ansprüche 1 bis 5, **dadurch gekennzeichnet, dass** das Bleisalz in Schritt (f) Bleihydroxidacetat ist.

7. Verfahren nach einem der Ansprüche 1 bis 6, **dadurch gekennzeichnet, dass** in Schritt (g) die Extraktion mindestens fünf mal erfolgt.

8. Verfahren nach einem der Ansprüche 1 bis 7, **dadurch gekennzeichnet, dass** in Schritt (i) die erhaltene Lösung mit einem Gemisch aus Methylethylketon und Ethanol in einem Verhältnis von 6:4 (G/G) extrahiert wird.

## Revendications

1. Un procédé de préparation d'un extrait de Ginkgo biloba à teneur réduite à hydrocarbures aromatiques polycycliques **caractérisé en ce que**
(a) on extrait des feuilles fraîches ou séchées de Ginkgo biloba (la drogue) avec de l'acétone aqueuse, d'un alcanol en C₁ à C₃ aqueux ou du méthanol à une température d'environ 40 à 100°C,
(b) on sépare le solvant organique de l'extrait pour la plus grande partie jusqu'à une teneur maximum de 2 % en poids et on peut ajouter de l'eau pendant les derniers seuils de distillation ;
(c) on dilue la solution résiduelle aqueuse concentrée avec de l'eau à une teneur en matière solide de 5 à 25 % en poids ; on refroidit sous agitation à une température inférieure à 6°C ; on laisse pour au moins 1 heure à cette température et on élimine le précipité obtenu consistant en des composants lipophiles qui ne sont pas bien solubles dans l'eau ;
(d) on ajoute du sulfate d'ammonium à la solution résiduelle aqueuse et on extrait la solution formée avec de la méthyléthylcétone ou un mélange de la méthyléthylcétone et de l'acétone,
(e) on concentre l'extrait obtenu à une teneur en matière solide de 50 à 70 % en poids et on dilue le concentré ainsi obtenu avec de l'eau et de l'éthanol pour obtenir une solution qui contient 50 % en poids d'eau et 50 % en poids d'éthanol à une teneur en matière solide d'environ 10 % en poids ;
une filtration est réalisée et le filtrat est traité à (f) ;
(f) à la solution ainsi obtenue on ajoute une solution aqueuse d'un sel de plomb jusqu'à un changement de couleur de brun à ambre et on élimine le précipité formé, ou on utilise un polyamide au lieu du sel de plomb ;
(g) on extrait la solution résiduelle hydro-alcoolique avec de l'heptane pour éliminer aussi les composés d'alkylphénol ;
(h) on concentre la solution résiduelle hydro-alcoolique sous pression réduite à une teneur d'éthanol d'environ 5 % en poids et on ajoute du sulfate d'ammonium jusqu'à une teneur de 20 % en poids ;
(i) on extrait la solution obtenue avec un mélange de méthyléthylcétone et d'éthanol dans des proportions en poids de 8 :2 à 5:5 ;
(k) on sèche la phase organique obtenue avec ≤ 20 % en poids du sulfate d'ammonium et on concentre à une teneur en matière solide de 50 à 70 % en poids, on ajoute de l'éthanol jusqu'à une teneur en éthanol d'au moins 80 % en poids est obtenue, on laisse au moins 2 h à ≤ 12 °C et on filtre ;
(l) on concentre le filtrat obtenu sous pression réduite à une température maximum de 60 ° à 80 °C pour obtenir un extrait sec avec une teneur en eau inférieure à 5 % en poids.

2. Le procédé selon la revendication 1,
**caractérisé en ce qu'**
à l'étape (a) la température est 40 à 60 °C et/ou le rapport drogue/solvant est de 1 :4 à 1 :20.

3. Le procédé selon la revendication 1 ou 2,
**caractérisé en ce qu'**
à l'étape (b) le solvant organique est séparé de l'extrait à une teneur maximum de 1 % en poids.

4. Le procédé selon l'une quelconque des revendications 1 à 3,
**caractérisé en ce qu'**
on laisse pour moins 10 h à une température inférieure à 6 °C.

5. Le procédé selon l'une quelconque des revendications 1 à 4,
**caractérisé en ce qu'**
on ajoute environ 30 % en poids du sulfate d'ammonium à la solution résiduelle aqueuse et/ou on extrait la solution formée avec un mélange de la méthyléthylcétone et de l'acétone avec un rapport de mélange de 6 :4 (p/p) à l'étape (d).

6. Le procédé selon l'une quelconque des revendications 1 à 5,
**caractérisé en ce que**
le sel de plomb à l'étape (f) est l'hydroxyde acétate de plomb.

7. Le procédé selon l'une quelconque des revendications 1 à 6,
**caractérisé en ce que**
l'extraction de l'étape (g) est réalisée au moins 5 fois.

8. Le procédé selon l'une quelconque des revendications 1 à 7,
**caractérisé en ce qu'**
à l'étape (i) la solution obtenue est extraite avec un mélange de la méthyléthylcétone et de l'éthanol avec un rapport de mélange de 6 : 4 (p/p).
